# EUROPÄISCHE PATENTSCHRIFT

(11) **EP 3 297 591 B1**
(45) Veröffentlichungstag und Bekanntmachung des Hinweises auf die Patenterteilung: **25.01.2023**
(21) Anmeldenummer: 16723760.1
(22) Anmeldetag: 20.05.2016
(51) Int. Cl.: A61F 13/00, A61F 13/02

(54) **PFLASTER MIT BIELASTISCHER RÜCKSCHICHT**
PLASTER WITH BI-ELASTIC BACKING LAYER
PANSEMENT PRÉSENTANT UNE COUCHE ARRIÈRE BI-ÉLASTIQUE

(30) Priorität: 21.05.2015 DE 102015006599; 31.03.2016 DE 102016105889
(43) Veröffentlichungstag der Anmeldung: 28.03.2018
(73) Patentinhaber: AdhexPharma SAS, 21300 Chenôve (FR)
(72) Erfinder: KAFFL, Hubert, 83730 Fischbachau (DE); GEGENFURTNER, Klaus, 83707 Bad Wiessee (DE); RODLER, Stefanie, 83626 Valley (DE)
(74) Vertreter: Beckord & Niedlich Patentanwälte PartG mbB
(86) Internationale Anmeldenummer: PCT/EP2016/061365
(87) Internationale Veröffentlichungsnummer: WO 2016/185001

(56) Entgegenhaltungen:
- EP-A1- 1 462 121
- EP-B1- 1 009 393
- WO-A2-01/91718
- DE-A1- 3 408 050
- DE-A1-102006 019 293
- DE-A1-102006 022 971
- DE-A1-102014 007 650
- US-A- 4 727 868

## Beschreibung

Die Erfindung betrifft ein Pflaster mit einer bielastischen Rückschicht.

Pflaster weisen im Allgemeinen auf einer der Haut zugewandten sogenannten Applikationsseite eine Matrix-Schicht auf, welche einen Wirkstoff enthalten kann. Auf der der Haut abgewandten Seite ist die Matrix-Schicht mit einer Rückschicht versehen, während auf der bei einem bestimmungsgemäßen Einsatz der Applikationsseite des Pflasters vor dessen Benutzung eine entfernbare Schutzfolie die Matrix-Schicht vor Verschmutzungen schützt.

Bei Pflastern erfolgt ein Wirkstoffeintrag in die Haut eines Patienten durch Diffusion des Wirkstoffs entlang des Wirkstoffkonzentrationsgefälles, das zwischen der (wirkstoffhaltigen) Matrix-Schicht des Pflasters und dem vom Blutgefäßsystem durchsetzten Bereich der Haut besteht. Der mit einem Pflaster erzielbare Wirkstoffblutspiegel wird, abgesehen von der Auflagefläche der Speicherschichtanordnung bzw. Matrix auf der Haut des Patienten, im Wesentlichen so über das Konzentrationsgefälle des Wirkstoffs an der Grenzschicht Haut zu Speicherschichtanordnung bzw. Matrix bestimmt.

Im Zuge eines verbesserten Tragekomforts bzw. verbesserten Hafteigenschaften kann eine Rückschicht eines Pflasters elastisch ausgebildet sein. Pflaster mit elastischer oder bielastischer Rückschicht gehören zum Stand der Technik. Beispielsweise beschreibt US 4 780 168 A eine Wundbandage mit einer planaren Streckungscharakteristik im Bereich von 0,5 bis 10 pounds/inch.

DE 34 08 050 A1 betrifft ein medizinisches Verbandmaterial mit einem komprimierten Polyurethanschaum, einer dehnbaren Vliesstofflage und einer Klebstoffmasse.

Nach EP 1 009 393 B1 sind jedoch Materialien mit einer derartigen Dehnbarkeit nicht als Rückschicht für Pflaster geeignet. Entweder ist ihre Dehnbarkeit zu gering, so dass sich beim Tragen auf der Haut ein unangenehmes Fremdkörpergefühl einstellt und auch die Gefahr besteht, dass das Pflaster die Bewegungen der Haut nicht ausreichend mitmacht und sich ablösen kann (EP 1 009 393 B1, Vergleichsbeispiel 2). Sind die Materialien jedoch sehr dehnbar, so tritt der sogenannte Schüsselbildungs-Effekt auf (EP 1 009 393 B1, Vergleichsbeispiel 1). Denn bei der Pflasterherstellung wird zuerst ein bandförmiger Verbund aus bzw. mit den Materialien für Rückschicht, Matrix-Schicht und Schutzschicht hergestellt. Dabei kann das Material der Schutzschicht weniger elastisch sein als das Material der Rückschicht. Wird das bandförmige Material der Rückschicht auf das bandförmige Material der Schutzschicht derart aufgebracht, dass beide Bänder die Matrix-Schicht einschließen, so ist das bandförmige Material der Rückschicht infolge angelegter Spannung stärker gestreckt als das Material der Schutzschicht. Wird nun das erhaltene Laminat einem Stanzvorgang unterworfen, um auf dem Band des Schutzschicht-Materials aus dem Band des Rückschicht-Materials einzelne Pflaster zu stanzen, führt die nach dem Stanzen wirkende elastische Rückstellkraft dazu, dass sich in Bandrichtung gegenüberliegende Randbereiche der vereinzelten Pflaster zurückziehen, während quer zur Bandrichtung gegenüberliegende Randbereiche sich nach außen entspannen bzw. ausdehnen können, wobei sich die vereinzelten Pflaster unter Bildung einer Schüssel mehr oder minder aufwerfen können (EP 1 009 393 B1, Vergleichsbeispiel 1). Dieser unerwünschte Effekt ist umso ausgeprägter, je größer die herzustellenden Produkte sind. Insbesondere längliche Produkte fallen dann nicht plan an, sondern wölben sich infolge der in Längs- und Querrichtung unterschiedlichen Spannungen und Rückstellkräfte bananenförmig auf, was eine Weiterverarbeitung erschwert oder gar unmöglich macht.

Diese Wanderungen der Randbereiche lassen sich daran erkennen, dass sich die in Bandrichtung gegenüberliegenden Randbereiche jeder Rückschicht hinter die Stanzspur zurückziehen, die das Stanzwerkzeug im Material der Schutzschicht hinterlässt, und dass sich die in Querrichtung gegenüberliegenden Randbereiche über die Stanzspur hinaus schieben und sie verdecken können. Beim Stanzvorgang muss sichergestellt sein, dass die Rückschichten der einzelnen Pflaster einwandfrei aus dem bandförmigen Rückschicht-Material herausgestanzt werden, wobei das Stanzwerkzeug leicht in das bandförmige Schutzschicht-Material eindringt und dort eine die Rückschicht zumindest im Moment des Stanzvorgangs eng umlaufende Spur hinterlässt. Dieses Eindringen des Stanzwerkzeugs in die Schutzschicht ist für die mechanischen Eigenschaften der Schutzschicht vernachlässigbar, führt also nicht dazu, dass die Schutzschicht beim späteren Abtrennen an der Spur reißt.

So schlägt EP 1 009 393 B1 ein Pflaster mit einer wiederablösbaren Schutzschicht, einer haftklebenden Reservoirschicht und einer gegebenenfalls haftkleberbeschichteten Rückschicht aus einem unidirektional und vorzugsweise längselastischen Material vor, das eine Elastizität von mindestens 20 % aufweist.

Ein Pflaster mit einer Rückschicht mit planarer Streckungscharakteristik oder mit einer bielastischen Rückschicht ist jedoch insofern vorzuziehen, als es den Bewegungen der Haut besser folgt.

Allgemein betrifft die Erfindung nun eine bielastische Schicht, wobei
- die bieleastische Schicht eine Folie aus gegebenenfalls offenporigem Schaum umfasst oder daraus besteht, wobei
- die Folie mit einem Element versehen ist, das zumindest in Richtung des größeren Durchmessers der ovalen Schaumporen dehnungsmindernd bis dehnungssteif ist, wobei das Element in Form von dehnungsmindernden bis dehnungssteifen Fäden oder dehnungsmindernden bis dehnungssteifen Filamenten, als Gewebe, als Flechtwerk, als Gewirke oder als Folie ausgebildet ist, wobei
- die Bielastizität der Schicht dadurch gekennzeichnet ist, dass die Schicht in mindestens zwei voneinander verschiedenen Richtungen in nicht nur unwesentlichem Umfang elastisches Verhalten zeigt, und
wobei die Rückschicht ausgewählt ist aus einem Polyolefin und/oder einem Polyurethan, besonders bevorzugt einem Polyolefin und/oder Polyurethan-Schaummaterial.

Insbesondere betrifft die Erfindung ein Pflaster mit einer bielastischen Rückschicht, bei dem
- die bieleastische Rückschicht eine Folie aus gegebenenfalls offenporigem Schaum umfasst oder daraus besteht, wobei
- die Folie mit einem Element versehen ist, das zumindest in Richtung des größeren Durchmessers der ovalen Schaumporen dehnungsmindernd ist, wobei
- die Bielastizität der Rückschicht dadurch gekennzeichnet ist, dass die Rückschicht in mindestens zwei voneinander verschiedenen Richtungen in nicht nur unwesentlichem Umfang elastisches Verhalten zeigt.

Erfindungsgemäß wird die Schicht bzw. Rückschicht als Verbund vorgesehen, der
- eine Folie mit oder aus einem gegebenenfalls offenporigen Schaum und
- einem dehnungsmindernden Element
umfasst oder daraus besteht. Dieses Element kann flächig ausgebildet sein bzw. eine Fläche bilden oder es kann in einer Fläche vorgesehen sein, wobei die Fläche jeweils in der Ebene des Schaumes oder parallel dazu liegt.

Unter einem dehnungsmindernden Element wird auch ein dehnungssteifes Element verstanden. Das dehnungsmindernde Element reduziert die Dehnung bzw. Dehnbarkeit der Schicht bzw. Rückschicht zumindest in Richtung des größeren Durchmessers der ovalen Schaumporen. Der Grad der Reduktion der Dehnbarkeit kann bis zur Dehnungssteife der Schicht bzw. Rückschicht eingestellt werden.

So betrifft die Erfindung auch ein Pflaster
(a) mit einer obligatorischen gegebenenfalls haftkleber-beschichteten bielastischen Rückschicht (Overtape),
(b) mit einer obligatorischen Deckschicht,
(c1) mit einer haftklebenden Matrix-Schicht und einer fakultativen haftklebenden Klebemittel-Schicht oder
(c2) mit einer nicht-haftklebenden Matrix-Schicht und einer haftklebenden Klebemittel-Schicht und
(d) mit einer obligatorischen wiederablösbaren Schutzschicht, wobei
   - die bieleastische Rückschicht eine Folie aus gegebenenfalls offenporigem Schaum umfasst oder daraus besteht, wobei
   - die Folie mit einem Element versehen ist, das zumindest in Richtung des größeren Durchmessers der ovalen Schaumporen dehnungsmindernd bis dehnungssteif ist,
   - wobei sich das Element in dieser Richtung durch die Folie oder über die Folie erstrecken kann, wobei
   - die Bielastizität der Rückschicht dadurch gekennzeichnet ist, dass die Rückschicht in zwei verschiedenen Richtungen in nicht nur unwesentlichem Umfang elastisches Verhalten zeigt.

Eine Rückschicht im Sinne der vorliegenden Erfindung kann dabei aus einer oder mehreren Schichten, insbesondere auch Folienschichten, ausgebildet sein.

Um eine verbesserte Haftung eines Pflasters auf der Haut eines Anwenders zu gewährleisten, kann ein bevorzugtes Pflaster eine Rückschicht aufweisen, welches ein Overtape umfasst bzw. bei welchem die Rückschicht als Overtape ausgebildet ist, d. h. seitlich über die Ränder einer Matrix-Schicht übersteht. Ein solches Overtape kann vorzugsweise mehrschichtig ausgebildet sein und eine Overtape-Folie selbst sowie eine Kleberschicht aufweisen. Insbesondere umfasst ein Overtape eine wirkstofffreie Kleberschicht, welche beispielsweise zur verbesserten Haftung auf der Haut und/oder zur Haftung auf einer Matrix-Schicht oder gegebenenfalls einer Zwischenschicht dient. Auch kann eine wirkstofffreie Kleberschicht lediglich bereichsweise auf eine Overtape-Folie aufgebracht sein, wie beispielsweise im Bereich eines seitlich über eine Matrix-Schicht überstehenden Bereichs.

Eine Overtape-Folie umfasst bevorzugt ein Polymer ausgewählt aus der Gruppe umfassend Polyolefine, Olefin-Copolymerisate, Polyester, Co-Polyester, Polyamide, Co-Polyamide, Polyurethane und dergleichen umfasst. Als Beispiele für geeignete Materialien können Polyester und hiervon insbesondere Polyethylenterephthalate als auch Polycarbonate, Polyolefine wie z. B. Polyethylene, Polypropylene oder Polybutylene, Polyethylenoxide, Polyurethane, Polystyrole, Polyamide, Polyimide, Polyvinylacetate, Polyvinylchloride, Polyvinylidenchloride, Co-Polymerisate, wie beispielsweise Acrylnitril-Butadien-Styrol-Terpolymere, oder Ethylen-Vinylacetat-Copolymerisate genannt werden. Ein Material eines Overtapes ist erfindungsgemäß ausgewählt aus einem Polyolefin und/oder einem Polyurethan, besonders bevorzugt einem Polyolefin und/oder Polyurethan-Schaummaterial.

Eine haftklebende oder nicht-haftklebende Matrix-Schicht eines erfindungsgemäßen Pflasters kann als feste bis halbfeste Matrix-Schicht oder in Form einer flüssigen sogenannten Reservoir-Schicht ausgebildet sein und kann dabei ein Wirkstoffdepot bilden. Auch kann ein erfindungsgemäßes Pflaster eine Matrix-Schicht und eine Reservoir-Schicht umfassen. Bevorzugt umfasst das erfindungsgemäße Pflaster jedoch eine Matrix-Schicht.

Unter einem dehnungsmindernden bis dehnungssteifen Element wird erfindungsgemäß auch ein unelastisches Element verstanden und umgekehrt.

Das dehnungsmindernde bis dehnungssteife Element kann in Richtung des größeren Durchmessers der ovalen Poren dieselbe Ausdehnung oder Länge haben wie die Schaumfolie.

Die ovale Form der Poren eines gegebenenfalls offenporigen Schaums resultiert daher, dass das auszuschäumende Material bei der Schaumherstellung in einer Richtung (unidirektional) auf eine Unterlage aufgetragen wird, wobei sich die Poren (in Draufsicht) nicht kugelförmig, sondern in Auftragsrichtung leicht gedehnt bzw. oval bzw. eiförmig ausbilden. Der hergestellte Schaum weist in der Auftragsrichtung bzw. in Richtung des größeren Durchmessers der einzelnen ovalen Poren eine größere Elastizität auf als quer dazu. Das dehnungsmindernde bis dehnungssteife Element soll dazu dienen, den Schaum in dieser Richtung zu versteifen.

Die Schaumfolie kann
- in der Richtung des größeren Durchmessers der ovalen Poren mit einem dehnungsmindernden bis dehnungssteifen Element über- oder unterzogen sein, beispielsweise durch Kaschieren, oder
- in der Richtung des größeren Durchmessers der ovalen Poren von einem dehnungsmindernden bis dehnungssteifen Element durchzogen sein.

Bei dem dehnungsmindernden bis dehnungssteifen Element handelt es sich um dehnungsmindernde bis dehnungssteife Fäden oder dehnungsmindernde bis dehnungssteife Filamente, insbesondere Einzelfäden oder Einzelfilamente, ein Gewebe mit insbesondere dehnungsmindernden bis dehnungssteifen Kettfäden, ein Flechtwerk mit insbesondere dehnungsminderndem bis dehnungssteifem Flechtstrang, ein Gewirke oder eine Folie handeln. Gewirke und Folie sind zumindest in Richtung des größeren Durchmessers der ovalen Poren des Schaums dehnungsmindernd bis dehnungssteif.

Diese dehnungsmindernden bis dehnungssteifen Elemente können leitfähig sein. Sie können elektrisch leitend sein.

So betrifft eine Ausführungsform der Erfindung ein Pflaster
(a) mit einer obligatorischen gegebenenfalls haftkleber-beschichteten bielastischen Rückschicht (Overtape),
(b) mit einer obligatorischen Deckschicht,
(c1) mit einer haftklebenden Matrix-Schicht und einer fakultativen haftklebenden Klebemittel-Schicht oder
(c2) mit einer nicht-haftklebenden Matrix-Schicht und einer haftklebenden Klebemittel-Schicht und
(d) mit einer obligatorischen wiederablösbaren Schutzschicht, wobei
   - die bieleastische Rückschicht eine Folie aus gegebenenfalls offenporigem Schaum umfasst oder daraus besteht,
   - der mit dehnungsmindernden bis dehnungssteifen Fäden versehen ist, die in Richtung des größeren Durchmessers der ovalen Poren verlaufen, oder
   - der mit einem Gewebe versehen ist, dessen dehnungsmindernde bis dehnungssteife Kettfäden in Richtung des größeren Durchmessers der ovalen Poren verlaufen, oder
   - der mit einem Flechtwerk (Geflecht) versehen ist, dessen dehnungsmindernder bis dehnungssteifer Flechtstrang in Richtung des größeren Durchmessers der ovalen Poren verläuft, wobei
   - die Bielastizität der Rückschicht dadurch gekennzeichnet ist, dass die Rückschicht in zwei verschiedenen Richtungen in nicht nur unwesentlichem Umfang elastisches Verhalten zeigt, wobei
   - bei dehnungsmindernden bis dehnungssteifen Fäden die beiden voneinander verschiedenen Richtungen weder parallel zu den Fäden noch in Richtung senkrecht zu den Fäden verlaufen,
   - bei einem Gewebe die beiden voneinander verschiedenen Richtungen weder parallel zu den Kettfäden des Gewebes noch in Richtung senkrecht zu den Kettfäden verlaufen und
   - bei einem Flechtwerk die beiden voneinander verschiedenen Richtungen weder parallel zum Strang des Flechtwerks noch in Richtung senkrecht zum Flechtstrang verlaufen.

Da derartige erfindungsgemäße Pflaster eine Rückschicht mit einer erfindungsgemäß gerichteten Bielastizität aufweisen, kann für die Herstellung der Pflaster ein Band aus dem bielastischen Rückschicht-Material eingesetzt werden, ohne dass die aus dem Band separierten einzelnen Rückschichten schrumpfen, so dass der Schüsselbildungs-Effekt nicht auftritt. Durch die Bielastizität ist jedoch Tragekomfort gewährleistet.

So betrifft eine weitere Ausführungsform der Erfindung ein Pflaster
(a) mit einer obligatorischen gegebenenfalls haftkleber-beschichteten bielastischen Rückschicht,
(b1) mit einer haftklebenden Matrix-Schicht und einer fakultativen haftklebenden Klebemittel-Schicht oder
(b2) mit einer nicht-haftklebenden Matrix-Schicht und einer haftklebenden Klebemittel-Schicht und
(c) mit einer obligatorischen wiederablösbaren Schutzschicht, wobei
   - die bieleastische Rückschicht eine Folie aus gegebenenfalls offenporigem Schaum umfasst oder daraus besteht,
   - der mit dehnungsmindernden bis dehnungssteifen Fäden versehen ist, die in Richtung des größeren Durchmessers der ovalen Poren verlaufen, oder
   - der mit einem Gewebe versehen ist, dessen dehnungsmindernde bis dehnungssteife Kettfäden in Richtung des größeren Durchmessers der ovalen Poren verlaufen, oder
   - der mit einem Flechtwerk (Geflecht) versehen ist, dessen dehnungsmindernder bis dehnungssteifer Flechtstrang in Richtung des größeren Durchmessers der ovalen Poren verläuft, wobei
   - die Bielastizität der Rückschicht dadurch gekennzeichnet ist, dass die Rückschicht in zwei verschiedenen Richtungen in nicht nur unwesentlichem Umfang elastisches Verhalten zeigt, wobei
   - bei dehnungsmindernden bis dehnungssteifen Fäden die beiden voneinander verschiedenen Richtungen weder parallel zu den Fäden noch in Richtung senkrecht zu den Fäden verlaufen,
   - bei einem Gewebe die beiden voneinander verschiedenen Richtungen weder parallel zu den Kettfäden des Gewebes noch in Richtung senkrecht zu den Kettfäden verlaufen und
   - bei einem Flechtwerk die beiden voneinander verschiedenen Richtungen weder parallel zum Strang des Flechtwerks noch in Richtung senkrecht zum Flechtstrang verlaufen.

Da derartige erfindungsgemäße Pflaster wiederum eine Rückschicht mit einer erfindungsgemäß gerichteten Bielastizität aufweisen, kann für die Herstellung der Pflaster ein Band aus dem bielastischen Rückschicht-Material eingesetzt werden, ohne dass die aus dem Band separierten einzelnen Rückschichten schrumpfen, so dass der Schüsselbildungs-Effekt nicht auftritt. Durch die Bielastizität ist jedoch Tragekomfort gewährleistet.

Die bielastische Schicht bzw. die Rückschicht eines erfindungsgemäßen Pflasters kann also u.a. mit dehnungsmindernden bis dehnungssteifen Fäden versehen sein, bei denen es sich um einzelne Fäden oder Filamente handelt. Einzel-Fäden oder Einzel-Filamente müssen kein Gewebe und keinen Strang bilden.

Eine Schicht bzw. Rückschicht ist insbesondere dann bielastisch, wenn sich ihre Reißdehnung in zwei verschiedenen Richtungen um nicht mehr als einen Faktor 2 unterscheidet und wenn sich ihr Elastizitätsmodul in zwei verschiedenen Richtungen um nicht mehr als einen Faktor 2 unterscheidet.

Eine Schicht bzw. Rückschicht zeigt in nicht nur unwesentlichem Umfang elastisches Verhalten, wenn ihre Reißdehnung mindestens 30 Prozent, vorzugsweise mehr als 30 Prozent und insbesondere mindestens 60 Prozent beträgt und/oder wenn nach einer Dehnung der Rückschicht auf 12/10 (zwölf Zehntel) ihrer ursprünglichen linearen Abmessung und Wegfall der dehnenden Kraft bzw. Spannung höchstens 11/10 (elf Zehntel) ihrer ursprünglichen linearen Abmessung verbleiben.

Beispielsweise gemäß EP 1 009 393 B1 ([0012] und [0013]) lässt sich Elastizität nach DIN 60 000, DIN 61 632 (April 1985) oder DIN 61 632 (Dezember 2009) messen.

In DIN 61 632:1985-04 (DIN 61 632 in der Fassung von April 1985) oder DIN 61 632:2009-12 (DIN 61 632 in der Fassung von Dezember 2009) ist eine Messvorschrift zur Bestimmung der Dehnung epsilon_ges für Idealbinden angegeben, die auch auf die erfindungsgemäß verwendete bielastische Rückschicht anwendbar ist.

Die gemäß DIN 61 632:1985-04 oder DIN 61 632:2009-12 gemessene Dehnung epsilon_ges der bielastischen Schicht bzw. Rückschicht sollte in zwei voneinander verschiedenen Richtungen mindestens 10 % betragen. Eine Dehnung bzw. Dehnbarkeit von weniger als 5 % wird als unelastisch angesehen. Die Dehnung epsilon_ges kann in den zwei voneinander verschiedenen Richtungen dieselbe sein. Die Dehnung epsilon_ges kann aber auch in den beiden voneinander verschiedenen Richtungen verschieden sein.

So kann die Dehnung epsilon_ges in mindestens einer dieser beiden voneinander verschiedenen Richtungen oder in beiden Richtungen mindestens 30 % betragen (jeweils gemessen nach DIN 61632).

So kann die Dehnung epsilon_ges der bielastischen Schicht bzw. Rückschicht ferner in mindestens einer der beiden voneinander verschiedenen Richtungen oder in beiden Richtungen 50 bis 75 % betragen, insbesondere 100 bis 125 %, vorzugsweise 150 bis 200 %, besonders bevorzugt 250 bis 300 % und vor allem 400 bis 500 % (jeweils gemessen nach DIN 61632).

Bei einem erfindungsgemäßen Pflaster können die zwei voneinander verschiedenen Richtungen sowohl in Richtung des größeren Durchmessers der ovalen Schaumporen als auch senkrecht zu dieser Richtung schräg oder geneigt sein.

Gegenüber der Richtung des größeren Durchmessers der ovalen Schaumporen oder quer dazu kann diese Neigung für mindestens eine der beiden voneinander verschiedenen Richtungen bis 45 Grad betragen. So können die beiden voneinander verschiedenen Richtungen in Bezug auf die Richtung des größeren Durchmessers der ovalen Schaumporen und quer dazu diagonal verlaufen.

In anderen Richtungen kann die bielastische Schicht bzw. Rückschicht unelastisches Verhalten zeigen.

Bei einer erfindungsgemäßen Schicht bzw. bei einem erfindungsgemäßen Pflaster können die zwei voneinander verschiedenen Richtungen
- bei einer Schicht oder Rückschicht mit dehnungsmindernden bis dehnungssteifen Fäden sowohl zur Richtung des Fadenverlaufs als auch senkrecht zu dieser Richtung schräg oder geneigt sein,
- bei einer Schicht oder Rückschicht mit Gewebe sowohl zur Richtung der Kette als auch senkrecht zu dieser Richtung schräg oder geneigt sein und
- bei einer Schicht oder Rückschicht mit einem Flechtwerk (Geflecht) sowohl zum Flechtstrang als auch senkrecht zu dieser Richtung schräg oder geneigt sein.

In anderen Richtungen kann die bielastische Schicht bzw. Rückschicht unelastisches Verhalten zeigen.

Gegenüber dem Verlauf von Fäden oder quer dazu kann diese Neigung für mindestens eine der beiden voneinander verschiedenen Richtungen bis 45 Grad betragen. So können die beiden voneinander verschiedenen Richtungen in Bezug auf die Kette und quer dazu diagonal verlaufen.

Gegenüber der Kette eines Gewebes oder quer dazu kann diese Neigung für mindestens eine der beiden voneinander verschiedenen Richtungen bis 45 Grad betragen. So können die beiden voneinander verschiedenen Richtungen in Bezug auf die Kette und quer dazu diagonal verlaufen.

Gegenüber dem Strang eines Geflechts oder quer dazu kann diese Neigung für mindestens eine der beiden voneinander verschiedenen Richtungen bis 45 Grad betragen. So können die beiden voneinander verschiedenen Richtungen in Bezug auf den Strang und quer dazu diagonal verlaufen.

Bei diesen Ausführungsformen kann die Schicht bzw. Rückschicht in Richtung
- parallel und quer zu den dehnungsmindernden bis dehnungssteifen Fäden oder
- parallel und quer zur Kette eines dehnungsmindernden bis dehnungssteifen Gewebes oder
- parallel und quer zum Strang eines dehnungsmindernden bis dehnungssteifen Flechtwerks unelastisches Verhalten zeigen, während die Dehnung epsilon_ges in zwei anderen und voneinander unabhängigen Richtungen mindestens 10 % betragen kann, beispielsweise 50 bis 75 %, insbesondere 100 bis 125 %, vorzugsweise 150 bis 200 %, besonders bevorzugt 250 bis 300 % und vor allem 400 bis 500 % (jeweils gemessen nach DIN 61632).

Erfindungsgemäße Pflaster mit diesen Merkmalen bieten vollen Tragekomfort, da sie bielastisch sind.

Bei dem erfindungsgemäßen Pflaster kann die bielastische Rückschicht die Matrix-Schicht samt Deckschicht auf der Schutzschicht allseitig abdecken oder die bielastische Rückschicht kann die Matrix-Schicht auf der Schutzschicht bedecken oder allseitig abdecken.

Das erfindungsgemäße Pflaster kann ein Schutzpflaster, ein Wundpflaster, ein kosmetisches Pflaster oder ein transdermales und insbesondere ein therapeutisches Pflaster sein. Als kosmetisches Pflaster oder als transdermales und insbesondere therapeutisches Pflaster kann die Matrix-Schicht Wirkstoffe enthalten.

Die vorliegende Erfindung betrifft somit auch die medizinische, tiermedizinische und/oder kosmetische Verwendung der erfindungsgemäßen Pflaster zur Abgabe von Wirkstoffen an und gegebenenfalls durch die Haut eines menschlichen oder tierischen Körpers und/oder an eine Umgebung um das Pflaster.

Bevorzugte Ausführungsformen eines erfindungsgemäßen Pflasters umfassen, wie erwähnt, eine zumindest einen Wirkstoff enthaltende Wirkstoffmatrix und/oder -reservoir. Bestimmte Ausführungsformen können auch mehr als eine wirkstoffhaltige Matrix bzw. mehr als ein wirkstoffhaltiges Reservoir umfassen, wobei die verwendeten Wirkstoffe gleich oder unterschiedlich sein können und in unterschiedlicher Konzentration vorliegen können.

Schließlich müssen nicht alle Schichten des erfindungsgemäßen Pflasters einen Wirkstoff enthalten, sondern das Pflaster kann zusätzlich zur zumindest einen wirkstoffhaltigen Matrixschicht eine oder mehrere wirkstofffreie Schichten aufweisen.

Die erfindungsgemäßen Pflaster sind zur Applikation von grundsätzlich allen Wirkstoffen oder Wirkstoffkombinationen geeignet. Unter einem Wirkstoff im Sinne der vorliegenden Erfindung werden dabei eine pharmazeutisch wirksame Substanz oder auch ein kosmetischer Wirkstoff und/oder Zusatzstoff und/oder Nährstoff oder Nahrungsergänzungsmittel und/oder ätherisches Öl verstanden.

Das erfindungsgemäße Pflaster gibt den Wirkstoff aus der wirkstoffhaltigen Matrix-Schicht an die Haut ab, wobei zumindest ein Teil des Wirkstoffs systemisch aufgenommen werden kann. Das Pflaster kann daher auch zur dermalen Abgabe eines Wirkstoffs eingesetzt werden, wie beispielsweise zur Lokalanästhesie der Haut. Des Weiteren kann ein Pflaster im Sinne der vorliegenden Erfindung auch einen Duftstoff, wie beispielsweise ein ätherisches Öl aus einer Matrix, abgeben. Vorteilhaft kann der Duftstoff hierbei im Wesentlichen durch eine poröse bzw. offenporige Rückschicht in die Umgebung abgegeben werden.

Die Wirkstoffe können in verschiedenen Formen in der Matrix-Schicht enthalten sein, je nachdem, welche Form die optimalen Abgabeeigenschaften des Wirkstoffs aus dem Pflaster ergibt. Im Falle von pharmazeutisch wirksamen Substanzen können diese in Form der freien Base oder Säure oder in Form von Salzen, Estern oder anderen pharmakologisch annehmbaren Derivaten oder als Komponenten von molekularen Komplexen vorliegen.

Die bielastische Rückschicht kann wirkstoffundurchlässig sein.

Der Schaum der bielastischen Schicht oder Rückschicht kann aus vernetztem Polyolefin bestehen und kann beispielsweise von Sekisui Alveo (Luzern, Schweiz) bezogen werden.

Die Fäden oder das Gewebe oder das Flechtwerk der bielastischen Schicht oder Rückschicht können aus Polyurethan oder aus Polyester bestehen, beispielsweise aus Polyethylenterephthalat. Diese Fäden, das Gewebe oder das Flechtwerk können elektrisch leitend sein.

Das Flächengewicht der bielastischen Schicht oder Rückschicht kann von 40 bis 250 g/m², insbesondere 70 bis 140 g/m², vorzugsweise 100 bis 140 g/m² und besonders bevorzugt 120 bis 140 g/m² betragen.

Die bielastische Schicht oder Rückschicht kann eine Dicke von 0,01 bis 1,00 mm, insbesondere 0,05 bis 0,50 mm und insbesondere 0,07 bis 0,10 mm aufweisen, gemessen jeweils vorzugsweise ungestreckt.

Sofern das erfindungsgemäße Pflaster zusätzlich zur Rückschicht eine Deckschicht aufweist, kann das Material der Deckschicht vorzugsweise wirkstoffundurchlässig sein. Ferner kann es dasselbe Material wie das der wiederablösbaren Schutzschicht sein.

Gemäß einer bevorzugten Ausführungsform sind die bielastische Schicht oder Rückschicht zumindest teilweise transparent ausgebildet. Eine solche zumindest teilweise transparente Schicht oder Rückschicht ist durchsichtig bis durchscheinend, jedoch nicht opak und ist durchlässig für elektromagnetische Wellen, insbesondere von Licht.

Eine zumindest teilweise transparente bielastische Schicht oder Rückschicht eines solchen Pflasters weist vorteilhaft eine Transmission von mindestens 5 %, bevorzugt von mindestens 10 %, besonders bevorzugt von mindestens 25 %, insbesondere von 50 %, insbesondere bevorzugt von mindestens 75 %, auf. Dabei kann die Schicht oder Rückschicht Bereiche aufweisen, welche nicht transparent sind.

Insofern die bielastische Schicht oder Rückschicht eines erfindungsgemäßen Pflasters aus einer oder mehreren Folienschichten ausgebildet ist, kann zumindest eine der Folienschichten zumindest teilweise transparent ausgebildet sein.

Gemäß einer besonders bevorzugten Ausführungsform befindet sich zwischen der Matrix-Schicht und der bielastischen Schicht oder Rückschicht eine Informationsschicht. Bevorzugt ist eine solche Informationsschicht in Form einer Bedruckung ausgebildet, welche auf der der Matrix-Schicht zugewandten Seite der bielastischen Schicht oder Rückschicht aufgebracht wird.

Gemäß einer weiter bevorzugten Ausführungsform kann ein erfindungsgemäßes Pflaster zwischen der Matrix-Schicht und der bielastischen Schicht oder Rückschicht eine sogenannte Zwischenschicht aufweisen. Eine solche Zwischenschicht kann dabei einen oder mehrere Haftkleber und eine oder mehrere Folien, sogenannte Zwischenfolien, aufweisen. Bevorzugt ist eine Zwischenschicht okklusiv, d. h. als Sperrschicht für beispielsweise den Durchtritt von Wirkstoffen, ausgebildet.

Wenn das erfindungsgemäße Pflaster eine Zwischenschicht aufweist, ist besonders bevorzugt eine Informationsschicht zwischen der bielastischen Schicht oder Rückschicht und der Zwischenschicht eingebracht. Ganz besonders bevorzugt ist die Informationsschicht dabei eine Bedruckung, welche auf der der bielastischen Schicht oder Rückschicht zugewandten Seite der Zwischenschicht aufgebracht wird. Alternativ oder zusätzlich kann eine Informationsschicht aber auch auf der der Matrix-Schicht zugewandten Seite der bielastischen Schicht oder Rückschicht aufgebracht sein. In diesem Fall kann eine Informationsschicht, beispielsweise eine Bedruckung, bevorzugt spiegelverkehrt aufgebracht sein. Das gleiche gilt, wenn die Informationsschicht auf einer der Matrix-Schicht zugewandten Seite einer Zwischenschicht aufgebracht wird, was insbesondere möglich ist, wenn die besagte Zwischenschicht auch zumindest teilweise transparent ist.

Insbesondere basiert eine bevorzugte Zwischenschicht auf einem Polymer ausgewählt aus der Gruppe umfassend Polyolefine, Olefin-Copolymerisate, Polyester, Co-Polyester, Polyamide, Co-Polyamide, Polyurethane und dergleichen. Als Beispiele für geeignete Materialien können Polyester und hiervon insbesondere Polyethylenterephthalate als auch Polycarbonate genannt werden, Polyolefine wie z. B. Polyethylene, Polypropylene oder Polybutylene, Polyethylenoxide, Polyurethane, Polystyrole, Polyamide, Polyimide, Polyvinylacetate, Polyvinylchloride, Polyvinylidenchloride, Co-Polymerisate, wie beispielsweise Acrylnitril-Butadien-Styrol-Terpolymere, oder Ethylen-Vinylacetat-Copolymerisate.

Bei dem Material der wiederablösbaren Schutzschicht bzw. Abziehfolie eines erfindungsgemäßen Pflasters kann es sich um Polyethylen (PE), Polypropylen (PP), Polyester, insbesondere Polyethylenterephthalat, ferner um Polyurethan oder Polyvinylchorid handeln.

Die wiederablösbare Schutzschicht sollte die Rückschicht allseitig überragen.

Schließlich kann die Konfiguration des Pflasters der vorliegenden Erfindung jeder Form oder Größe entsprechen, die notwendig oder wünschenswert ist. Zum Beispiel kann ein vorteilhaftes Pflaster eine Größe im Bereich von 1 bis 100 cm², bevorzugt im Bereich von 2,5 bis 60 cm², besonders bevorzugt im Bereich von 5 bis 40 cm², insbesondere im Bereich von 10 bis 25 cm² aufweisen.

### Beispiele 1 bis 2

Es wurden auf ein Band eines einseitig silikonisierten Schutzschicht-Materials (Polyethylenterephthalat; z.B. Primeliner PET 75 mikro m 1S) Pflasterkerne gespendet. Die Pflasterkerne waren ein Verbund aus einer wirkstoffundurchlässigen Trennschicht bzw. Deckschicht (Polyethylenterephthalat; z.B. Primeliner PET 75 mikro m 1S) und aus einer Matrix-Schicht (z.B. DuroTak 6911A). Danach wurde ein Band aus einem bielastischen Rückschicht-Material (Band aus vernetztem Polyolefin-Schaum mit unelastischen Nylonfilamenten, eingebettet in Richtung des größeren Durchmessers der ovalen Poren; in Längs- und Querrichtung unelastisch, Dehnung in Richtung der beiden Diagonalen größer gleich 10 % bzw. größer gleich 35 %) auf das Band des Schutzschicht-Materials mit den darauf gespendeten Pflasterkernen aufkaschiert. Aus dem Band des aufkaschierten bielastischen Rückschicht-Materials wurden einzelne Rückschichten derart gestanzt, dass die Rückschichten die Trennschichten mit ihren Matrix-Schichten auf dem Band des Schutzschicht-Materials allseitig abdeckten. Schließlich wurden aus dem Band des Schutzschicht-Materials einzelne Schutzschichten gestanzt, die die Rückschichten allseitig überragten.

Die Distanz der sich gegenüberliegenden Ränder der Rückschichten wichen um weniger als 0,5 % von den zu ihnen parallelen Abschnitten der Kerbe ab, die das Stanzwerkzeug in den Schutzschichten hinterlassen hatte.

### Vergleichbeispiel 1

Es wurde EP 1 009 393 B1 Vergleichsbeispiel 1 nachgearbeitet. Etwa 50 % der hergestellten Pflaster zeigten entweder einen Schüsselbildungseffekt oder die bielastischen Rückschichten hatten sich in Prozessrichtung um mehr als 5 % von der Stanzkerbe zurückgezogen.

### Tests 1 bis 2

Es wurde die Längsdehnung eines Rückschicht-Materials (Firma Karl Otto Braun/Wolfstein; Polyethylenterephthalat; Flächengewicht 130 +/- 20 g/m²; PET-Gewebe; TL 053/11-C) mit einer Zugkraftprüfmaschine (Firma Zwick/Ulm) gemäß DIN 61632 bestimmt.
Maschine Z 0.5
Kraftaufnehmer: XforceP, 500 N, G.-Nr. 450
Teststreifenbreite 30 mm; b null 30 mm
Einspannlänge bei Startposition
   Test 1: 200,06 mm
   Test: 2 199,70 mm
Zielposition im Abschnitt/Belastung 10 N/cm
Geschwindigkeit im Abschnitt 500 mm/min
Haltezeit im Abschnitt 60 s
Vorkraft 10 mn (keine Haltezeit der Vorkraft, Kraft wird bei Erreichen nicht genullt)
Dehnung absolut (Bindenlänge gedehnt - Einspannlänge) 2
   Test 1: 105,97 mm
   Test 2: 104,33 mm
epsilon ges
   Test 1: 52,97 %
   Test 2: 52,24 %

## Patentansprüche

1. Pflaster mit einer bielastischen Rückschicht, bei dem
- die bieleastische Rückschicht eine Folie aus gegebenenfalls offenporigem Schaum umfasst oder daraus besteht, wobei
- die Folie mit einem Element versehen ist, das zumindest in Richtung des größeren Durchmessers der ovalen Schaumporen dehnungsmindernd bis dehnungssteif ist, wobei das Element in Form von dehnungsmindernden bis dehnungssteifen Fäden oder dehnungsmindernden bis dehnungssteifen Filamenten, als Gewebe, als Flechtwerk, als Gewirke oder als Folie ausgebildet ist, wobei
- die Bielastizität der Rückschicht **dadurch gekennzeichnet ist, dass** die Rückschicht in zwei voneinander verschiedenen Richtungen in nicht nur unwesentlichem Umfang elastisches Verhalten zeigt, und
wobei die Rückschicht ausgewählt ist aus einem Polyolefin und/oder einem Polyurethan, besonders bevorzugt einem Polyolefin und/oder Polyurethan-Schaummaterial.

2. Pflaster nach Anspruch 1
(a) mit einer obligatorischen gegebenenfalls haftkleber-beschichteten bielastischen Rückschicht (Overtape),
(b) mit einer fakultativen Deckschicht,
(c1) mit einer haftklebenden Matrix-Schicht und einer fakultativen haftklebenden Klebemittel-Schicht oder
(c2) mit einer nicht-haftklebenden Matrix-Schicht und einer haftklebenden Klebemittel-Schicht und
(d) mit einer obligatorischen wiederablösbaren Schutzschicht, wobei
- die bieleastische Rückschicht eine Folie aus gegebenenfalls offenporigem Schaum umfasst oder daraus besteht, wobei
- die Folie mit einem Element versehen ist, das zumindest in Richtung des größeren Durchmessers der ovalen Schaumporen dehnungsmindernd bis dehnungssteif ist, wobei das Element in Form von dehnungsmindernden bis dehnungssteifen Fäden oder dehnungsmindernden bis dehnungssteifen Filamenten, als Gewebe, als Flechtwerk, als Gewirke oder als Folie ausgebildet ist, wobei
- die Bielastizität der Rückschicht **dadurch gekennzeichnet ist, dass** die Rückschicht in zwei voneinander verschiedenen Richtungen in nicht nur unwesentlichem Umfang elastisches Verhalten zeigt, und
wobei die Rückschicht ausgewählt ist aus einem Polyolefin und/oder einem Polyurethan, besonders bevorzugt einem Polyolefin und/oder Polyurethan-Schaummaterial.

3. Pflaster nach Anspruch 1 oder 2, wobei es sich bei den dehnungsmindernden bis dehnungssteifen Fäden oder dehnungsmindernden bis dehnungssteifen Filamenten um Einzelfäden oder Einzelfilamente, bei dem Gewebe um dehnungsmindernde bis dehnungssteife Kettfäden oder bei dem Flechtwerk um einen dehnungsmindernden bis dehnungssteifen Flechtstrang handelt.

4. Pflaster nach Anspruch 1, 2 und/oder 3, bei dem die zwei voneinander verschiedenen Richtungen sowohl in Richtung des größeren Durchmessers der ovalen Schaumporen als auch senkrecht zu dieser Richtung schräg oder geneigt sind.

5. Pflaster nach mindestens einem der Ansprüche 1, 2, 3 und 4, bei dem die zwei voneinander verschiedenen Richtungen
- bei einer mit Fäden versehenen Rückschicht sowohl in Richtung der Fäden als auch zur Richtung senkrecht dazu schräg oder geneigt sind,
- bei einer mit Gewebe versehenen Rückschicht sowohl zum Schuss als auch zur Richtung senkrecht dazu schräg oder geneigt sind und
- bei einer mit Flechtwerk versehenen Rückschicht sowohl zum Flechtstrang als auch zur Richtung senkrecht dazu schräg oder geneigt sind.

6. Pflaster nach mindestens einem der Ansprüche 1, 2, 3, 4 und 5, bei dem die Elastizität der bielastischen Rückschicht in zwei voneinander verschiedenen Richtungen mindestens 10 % beträgt, vorzugsweise in mindestens einer der beiden voneinander verschiedenen Richtungen oder in beiden Richtungen mindestens 30 % beträgt (jeweils gemessen nach DIN 61632).

7. Pflaster nach Anspruch 6, bei dem die Elastizität der bielastischen Rückschicht in mindestens einer der beiden voneinander verschiedenen Richtungen oder in beiden Richtungen 50 bis 75 % beträgt, insbesondere 100 bis 125 %, vorzugsweise 150 bis 200 %, besonders bevorzugt 250 bis 300 % und vor allem 400 bis 500 % (jeweils gemessen nach DIN 61632).

8. Pflaster nach mindestens einem der Ansprüche 1, 2, 3, 4, 5, 6 und 7, bei dem die bielastische Rückschicht wirkstoffundurchlässig ist.

9. Pflaster nach mindestens einem der Ansprüche 1, 2, 3, 4, 5, 6, 7 und 8 mit einer bielastischen Rückschicht,
- auf die die dehnungsmindernden bis dehnungssteifen Fäden oder das dehnungsmindernde bis dehnungssteife Gewebe oder das dehnungsmindernde bis dehnungssteife Flechtwerk aufkaschiert oder
- in die die dehnungsmindernden bis dehnungssteifen Fäden oder das dehnungsmindernde bis dehnungssteife Gewebe oder das dehnungsmindernde bis dehnungssteife Flechtwerk eingebettet sind.

10. Pflaster nach mindestens einem der Ansprüche 1, 2, 3, 4, 5, 6, 7, 8 und 9 mit einer bielastischen Rückschicht eines Flächengewichts von 40 bis 250, insbesondere 70 bis 140, vorzugsweise 100 bis 140 und besonders bevorzugt 120 bis 140 g/m².

11. Pflaster nach mindestens einem der Ansprüche 1, 2, 3, 4, 5, 6, 7, 8, 9 und 10 mit einer bielastischen Rückschicht einer Dicke von 0,01 bis 1,00, insbesondere 0,05 bis 0,50 und insbesondere 0,07 bis 0,10 mm, gemessen jeweils vorzugsweise ungestreckt.

12. Pflaster nach einem der vorhergehenden Ansprüche, wobei die bielastische Rückschicht zumindest teilweise transparent ausgebildet ist.

13. Pflaster nach einem der vorhergehenden Ansprüche, wobei die bielastische Rückschicht auf einer zur Matrix-Schicht gewandten Seite eine Informationsschicht, vorzugsweise eine Bedruckung, aufweist.

14. Pflaster nach einem der vorhergehenden Ansprüche, wobei sich zwischen der bielastischen Rückschicht und der Matrix-Schicht zumindest eine Zwischenschicht befindet.

15. Pflaster nach Anspruch 14, wobei eine Informationsschicht auf der Zwischenschicht aufgebracht, vorzugsweise aufgedruckt, ist.

16. Pflaster nach einem der vorhergehenden Ansprüche zur medizinischen, tiermedizinischen und/oder kosmetischen Anwendung.

## Claims

1. Plaster with a bielastic backing layer, in which
- the bielastic backing layer comprises or consists of a film of optionally open-pored foam, wherein
- the film is provided with an element that is stretch-reducing to stretch-resistant, at least in the direction of the larger diameter of the oval foam pores, the element being designed in the form of stretch-reducing to stretch-resistant threads or stretch-reducing to stretch-resistant filaments, as a woven fabric, as a braid, as a knitted fabric or as a foil, wherein
- the bielasticity of the backing layer is **characterized in that** the backing layer shows elastic behavior in two different directions to a not insignificant extent, and
wherein the backing layer is selected from a polyolefin and/or a polyurethane, particularly preferably a polyolefin and/or polyurethane foam material.

2. Plaster according to claim 1
(a) with an obligatory optionally pressure-sensitive adhesive-coated bielastic backing layer (overtape),
(b) with an optional cover layer,
(c1) having a pressure-sensitive adhesive matrix layer and an optional pressure-sensitive adhesive layer, or
(c2) with a non-pressure-sensitive adhesive matrix layer and a pressure-sensitive adhesive layer, and
(d) with a mandatory removable protective layer, wherein
- the bielastic backing layer comprises or consists of a film of optionally open-pored foam, wherein
- the film is provided with an element that is stretch-reducing to stretch-resistant, at least in the direction of the larger diameter of the oval foam pores, the element being designed in the form of stretch-reducing to stretch-resistant threads or stretch-reducing to stretch-resistant filaments, as a woven fabric, as a braid, as a knitted fabric or as a foil, wherein
- the bielasticity of the backing layer is **characterized in that** the backing layer shows elastic behavior in two different directions to a not insignificant extent, and
wherein the backing layer is selected from a polyolefin and/or a polyurethane, particularly preferably a polyolefin and/or polyurethane foam material.

3. Plaster according to claim 1 or 2, wherein the stretch-reducing to stretch-rigid threads or stretch-reducing to stretch-rigid filaments are single threads or single filaments, the fabric is stretch-reducing to stretch-rigid warp threads or the braiding is a stretch-reducing to stretch-rigid braided strand.

4. Plaster according to claim 1, 2 and/or 3, in which the two mutually different directions are oblique or inclined both in the direction of the larger diameter of the oval foam pores and perpendicular to this direction.

5. Plaster according to at least one of claims 1, 2, 3 and 4, wherein the two different directions
- in the case of a backing layer provided with threads, are oblique or inclined both in the direction of the threads and in the direction perpendicular thereto,
- in the case of a fabric backing, are oblique or inclined both to the weft and to the direction perpendicular thereto, and
- in the case of a braided backing, are oblique or inclined both to the braid strand and to the direction perpendicular thereto.

6. Plaster according to at least one of claims 1, 2, 3, 4 and 5, in which the elasticity of the bielastic backing layer in two different directions is at least 10 %, preferably in at least one of the two different directions or in both directions at least 30 % (each measured according to DIN 61632).

7. Plaster according to claim 6, in which the elasticity of the bielastic backing layer in at least one of the two directions which differ from one another or in both directions is 50 to 75 %, in particular 100 to 125 %, preferably 150 to 200 %, particularly preferably 250 to 300 % and above all 400 to 500 % (each measured according to DIN 61632).

8. Plaster according to at least one of claims 1, 2, 3, 4, 5, 6 and 7, in which the bielastic backing layer is impermeable to the active substance.

9. Plaster according to at least one of claims 1, 2, 3, 4, 5, 6, 7 and 8 with a bielastic backing layer,
- on which the stretch-reducing to stretch-rigid threads or the stretch-reducing to stretch-rigid fabric or the stretch-reducing to stretch-rigid braiding is laminated, or
- in which the stretch-reducing to stretch-rigid threads or the stretch-reducing to stretch-rigid fabric or the stretch-reducing to stretch-rigid braiding is embedded.

10. Plaster according to at least one of claims 1, 2, 3, 4, 5, 6, 7, 8 and 9 with a bielastic backing layer with an area weight of 40 to 250, in particular 70 to 140, preferably 100 to 140 and particularly preferably 120 to 140 g/m².

11. Plaster according to at least one of claims 1, 2, 3, 4, 5, 6, 7, 8, 9 and 10 with a bielastic backing layer having a thickness of 0.01 to 1.00, in particular 0.05 to 0.50 and in particular 0.07 to 0.10 mm, each measured preferably unstretched.

12. Plaster according to one of the preceding claims, wherein the bielastic backing layer is designed at least partially transparent.

13. Plaster according to one of the preceding claims, wherein the bielastic backing layer comprises an information layer on a side facing the matrix layer, preferably a printing.

14. Plaster according to one of the preceding claims, wherein there is at least one intermediate layer between the bielastic backing layer and the matrix layer.

15. Plaster according to claim 14, wherein an information layer is applied, preferably printed, to the intermediate layer.

16. Plaster according to one of the preceding claims for medical, veterinary and/or cosmetic use.

## Revendications

1. Pansement, pourvu d'une couche arrière biélastique, sur lequel
- la couche arrière biélastique comprend un film en une mousse, à pores ouverts le cas échéant ou en est constituée,
- le film est muni d'un élément, qui au moins en direction du plus grand diamètre des pores de mousse ovales est de réducteur d'allongement à rigide en allongement, l'élément étant conçu sous la forme de fils réducteurs d'allongement à rigides en allongement, ou de filaments réducteurs d'allongement à rigides en allongement, sous la forme d'un tissu, d'un tressage, d'un tricot ou d'un film,
- la biélasticité de la couche arrière étant **caractérisée en ce que** la couche arrière fait preuve dans deux directions différentes l'une de l'autre, dans une mesure pas seulement négligeable d'un comportement élastique, et
la couche arrière étant sélectionnée parmi une polyoléfine et/ou un polyuréthane, de manière particulièrement préférentielle, une polyoléfine et/ou une matière de mousse de polyuréthane.

2. Pansement selon la revendication 1
(a) pourvu d'une couche arrière biélastique (Overtape) obligatoire, recouverte le cas échéant d'un agent adhésif,
(b) pourvu d'une couche couvrante facultative,
(c1) pourvu d'une couche matricielle adhésive et d'une couche de colle adhésive facultative ou
(c2) pourvu d'une couche matricielle non adhésive et d'une couche de colle adhésive et
(d) pourvu d'une couche protectrice pelable obligatoire, où
- la couche arrière biélastique comprenant un film en une mousse, à pores ouverts le cas échéant ou en étant constituée, où
- le film étant muni d'un élément, qui au moins en direction du plus grand diamètre des pores de mousse ovales est de réducteur d'allongement à rigide en allongement, l'élément étant conçu sous la forme de fils réducteurs d'allongement à rigides en allongement, ou de filaments réducteurs d'allongement à rigides en allongement, sous la forme d'un tissu, d'un tressage, d'un tricot ou d'un film, où
- la biélasticité de la couche arrière étant **caractérisée en ce que** la couche arrière fait preuve dans deux directions différentes l'une de l'autre, dans une mesure pas seulement négligeable d'un comportement élastique, et
la couche arrière étant sélectionnée parmi une polyoléfine et/ou un polyuréthane, de manière particulièrement préférentielle, une polyoléfine et/ou une matière de mousse de polyuréthane.

3. Pansement selon la revendication 1 ou 2, les fils réducteurs d'allongement à rigides en allongement ou les filaments réducteurs d'allongement à rigides en allongement étant des fils individuels ou des filaments individuels, ceux dans le tissu étant des fils de chaine réducteurs d'allongement à rigides en allongement ou dans le tressage étant une tresse réductrice d'allongement à rigide en allongement.

4. Pansement selon la revendication 1, 2 et / ou 3, sur lequel, aussi bien dans la direction du plus grand diamètre des pores de mousse ovales qu'également à la perpendiculaire de cette direction, les deux directions différentes l'une de l'autre sont en oblique ou inclinées.

5. Pansement selon au moins l'une quelconque des revendications 1, 2, 3 et 4, sur lequel les deux directions différentes l'une de l'autre
- dans une couche arrière munie de fils, aussi bien dans la direction des fils qu'à la direction perpendiculaire à ceux-ci, sont obliques ou inclinées par rapport à celles-ci,
- dans une couche arrière munie de tissu, aussi bien par rapport à la trame qu'à la direction perpendiculaire à celle-ci, sont obliques ou inclinées par rapport à celles-ci, aussi bien par rapport à la trame qu'à la direction perpendiculaire à celles-ci, et
- dans une couche arrière munie d'un tressage, aussi bien par rapport à la tresse qu'à la direction perpendiculaire à celle-ci, sont obliques ou inclinées par rapport à celles-ci.

6. Pansement selon au moins l'une quelconque des revendications 1, 2, 3, 4 et 5, sur lequel, dans deux directions différentes l'une de l'autre, l'élasticité de la couche arrière biélastique s'élève à au moins 10 %, de préférence dans au moins l'une des deux directions différentes l'une de l'autre ou s'élève à au moins 30 % dans les deux directions (mesurés chaque fois selon la norme DIN 61632).

7. Pansement selon la revendication 6, sur lequel, dans au moins l'une des deux directions différentes l'une de l'autre ou dans le deux directions, l'élasticité de la couche arrière biélastique s'élève à de 50 à 75 %, notamment à de 100 à 125 %, de préférence, à de 150 à 200 %, de manière particulièrement préférentielle, à de 250 et 300 % et prioritairement, à de 400 à 500 % (mesurés chaque fois selon la norme DIN 61632).

8. Pansement selon au moins l'une quelconque des revendications 1, 2, 3, 4, 5, 6 et 7, sur lequel la couche arrière biélastique est imperméable aux principes actifs.

9. Pansement selon au moins l'une quelconque des revendications 1, 2, 3, 4, 5, 6, 7 et 8, pourvu d'une couche arrière biélastique,
- sur laquelle les fils réducteurs d'élasticité à rigides en élasticité ou le tissu réducteur d'élasticité à rigide en élasticité ou le tressage réducteur d'élasticité à rigide en élasticité sont contrecollés ou
- dans laquelle les fils réducteurs d'élasticité à rigides en élasticité ou le tissu réducteur d'élasticité à rigide en élasticité ou le tressage réducteur d'élasticité à rigide en élasticité sont incorporés.

10. Pansement selon au moins l'une quelconque des revendications 1, 2, 3, 4, 5, 6, 7, 8 et 9, pourvu d'une couche arrière biélastique d'un grammage de 40 à 250, notamment de 70 à 140, de préférence, de 100 à 140 et de manière particulièrement préférentielle, de 120 à 140 g/m².

11. Pansement selon au moins l'une quelconque des revendications 1, 2, 3, 4, 5, 6, 7, 8, 9 et 10, pourvu d'une couche arrière biélastique d'une épaisseur de 0,01 à 1,00, notamment de 0,05 à 0,50 et notamment de 0,07 à 0,10 mm, mesurés chaque fois de préférence à l'état non étiré.

12. Pansement selon l'une quelconque des revendications précédentes, la couche arrière biélastique étant conçue en étant au moins en partie transparente.

13. Pansement selon l'une quelconque des revendications précédentes, sur une face dirigée vers la couche matricielle, la couche arrière biélastique comportant une couche informative, de préférence une impression.

14. Pansement selon l'une quelconque des revendications précédentes, entre la couche arrière biélastique et la couche matricielle se trouvant au moins une couche intermédiaire.

15. Pansement selon la revendication 14, une couche informative étant appliquée, de préférence imprimée sur la couche intermédiaire.

16. Pansement selon l'une quelconque des revendications précédentes, destiné à des applications médicales, vétérinaires et/ou cosmétiques.
